# EUROPÄISCHE PATENTSCHRIFT

(11) **EP 1 021 519 B1**
(45) Veröffentlichungstag und Bekanntmachung des Hinweises auf die Patenterteilung: **18.12.2002**
(21) Anmeldenummer: 98954377.2
(22) Anmeldetag: 08.10.1998
(51) Int. Cl.: C11D 3/43, C11D 3/30, C11D 7/32

(54) **VERFAHREN ZUR REINIGUNG CHIRURGISCHER INSTRUMENTE**
PROCESS FOR CLEANING SURGICAL INSTRUMENTS
PROCEDE POUR LE NETTOYAGE D'INSTRUMENTS CHIRURGICAUX

(30) Priorität: 08.10.1997 DE 19744434
(43) Veröffentlichungstag der Anmeldung: 26.07.2000
(73) Patentinhaber: Chemische Fabrik Dr. Weigert GmbH & Co. KG., 20539 Hamburg (DE)
(72) Erfinder: STAFFELDT, Jürgen, D-21423 Winsen (DE); TIARKS, Petra, D-21031 Hamburg (DE)
(74) Vertreter: Glawe, Delfs, Moll & Partner
(86) Internationale Anmeldenummer: EP9806396
(87) Internationale Veröffentlichungsnummer: WO99018184

(56) Entgegenhaltungen:
- EP-A- 0 730 024
- WO-A-94/02179
- WO-A-99/15012
- GB-A- 2 053 954
- GB-A- 2 068 405
- GB-A- 2 204 321

## Beschreibung

Gegenstand der vorliegenden Erfindung ist die Verwendung eines Reinigungsmittelkonzentrats zur insbesondere maschinellen Reinigung medizinischer und/oder chirurgischer Instrumente und/oder Apparate sowie ein Verfahren zum Durchführen dieser Reinigung.

Chirurgische Instrumente sowie andere medizinische Geräte werden im Krankenhaus üblicherweise unter Verwendung alkalischer Reinigungsmittel maschinell gereinigt und anschließend chemisch oder thermisch desinfiziert. Solche stark alkalische Mittel können aggressiv gegenüber empfindlichen Oberflächen wirken. Mit Blut verunreinigte chirurgische Instrumente werden häufig unmittelbar nach ihrer Benutzung bspw. in eine aldehydhaltige Desinfektionslösung abgelegt und verbleiben darin zunächst, bis sie zum Reinigen in die Spülmaschine geräumt werden. Das Blut wird durch die Desinfektionsmittel koaguliert und die im Blut enthaltenen Eiweißbestandteile durch den aldehydischen Desinfektionswirkstoff denaturiert. Solche besonders hartnäckigen Blutrückstände sind häufig nur durch alkalisch-aktivchlorhaltige Reinigungsmittel zu entfernen. Die oxidierende Aktivchlorkomponente bewirkt die Zersetzung der denaturierten Eiweißbestandteile. Auch andere Bestandteile von Desinfektionsmitteln wie bspw. Jod können schwer zu entfernende Rückstände bilden.

Die alkalisch-aktivchlorhaltigen Reiniger weisen die Nachteile auf, daß sie kennzeichnungspflichtige Gefahrenstoffe enthalten, daß bei ihrer Handhabung besondere Vorsichtsmaßnahmen zum Schutz des Bedienungspersonals erforderlich sind und daß sie im Abwasser eine unerwünschte Umweltbelastung darstellen.

Aus US-A-4,243,546, EP-A-0 481 663 und EP-A-0 730 024 sind enzymhaltige Reinigungsmittel bekannt, die insbesondere Blutproteine enzymatisch abbauen können. Es wird dort vorgeschlagen, Triethanolamin zur Stabilisierung der Enzyme zu verwenden. Nachteilig an diesen Reinigern ist der hohe Preis der Enzyme, deren Temperaturempfindlichkeit sowie das Nachlassen der Enzymaktivität bei längerer Lagerung, insbesondere bei höheren Temperaturen.

Aus GB-A-2 068 405 und GB-A-2 204 321 sind stark alkalische Reiniger mit einem pH-Wert von 11 bis 12,5 bekannt, die aufgrund ihrer Alkalität agressiv gegenüber empfindlichen Oberflächen medizinischer und chirurgischer Instrumente sind und dennoch eine nicht hinreichende Reinigungswirkung gegenüber hartnäckigen Blutrückständen aufweisen.

WO-A-99/15012 (Stand der Technik nach Art. 54(3) EPÜ) offenbart ein Verfahren zur Reinigung und Desinfektion von medizinischen Instrumenten, bei dem eine wäßrige Reinigungs- und Desinfektionslösung verwendet wird, die einen Aldehyd sowie ein Ethanolamin enthält.

Der Erfindung liegt die Aufgabe zugrunde, ein Verfahren zum Reinigen von medizinischen und/oder chirurgischen Instrumenten und/oder Apparaten zu schaffen, bei dem die Nachteile der aktivchlorhaltigen und enzymatischen Reinigern nicht oder in geringerem Maße auftreten und das dennoch eine ausreichende Reinigungswirkung gewährleistet.

Die Erfindung löst diese Aufgabe durch die Merkmale der Ansprüche 1 und 12. Vorteilhafte Ausgestaltungen sind in den Unteransprüchen angegeben.

Zwar sind alkanolaminhaltige enzymatische Reiniger im Stand der Technik bereits bekannt. Doch wird dem Alkanolamin dort lediglich eine das Enzym stabilisierende Wirkung zugesprochen (US-A-4,243,546), das eigentliche reinigende Agens soll der enzymatische Wirkstoff sein bzw. Alkanolamin ist eine von mehreren Komponenten einer Reinigungsmittellösung in einem stark alkalischen Anwendungsbereich von pH 11 bis 12,5 (GB-A-2 068 405, GB-A-2 204 321) oder in einem ph-Bereich von 6 bis 10, wobei neben dem Alkanolamin mindestens ein Aldehyd und mindestens ein Tensiol weitere erforderliche Komponenten der Reinigungs- und Desinfektionslösung sind (WO-A-99/15012). Letzteres Dokument fällt unter Art. 54(3) EPÜ. Überraschenderweise hat sich aber nun gezeigt, daß ein Alkanolamin als Hauptwirkkomponente eines aldehydfreien Reinigungsmittels geeignet ist, im Rahmen einer maschinellen Reinigung angetrocknete, vorerhitzte oder mit bspw. aldehydischen Desinfektionswirkstoffen denaturierte Blutrückstände von chirurgischen Instrumenten einwandfrei zu entfernen. Auch die von Desinfektionsmitteln häufig herrührenden Jodrückstände bzw. deren Reaktionsprodukte mit Blutbestandteilen werden entfernt. Diese Reinigungswirkung ergibt sich trotz Verzicht auf im Stand der Technik als wesentlich angesehene Enzyme und weitere Reiniungs- und Desinfektionslösungsmittelbestandteile.

Vorzugsweise haben die verwendeten Alkanolamine folgende Struktur: 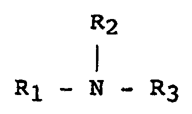 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

Besonders bevorzugt sind Mono-, Di- und/oder Triethanolamin.

Bei den erfindungsgemäß verwendeten Alkanolaminen handelt es sich um Basen. Das Reinigerkonzentrat weist in gebrauchsfertig verdünnter wäßriger Lösung einen pH-Wert von 6 bis 10, vorzugsweise 7 bis 9 auf. Es hat sich herausgestellt, daß überraschenderweise die an und für sich alkalischen Alkanolamine auch in diesem schwach sauren bis schwach alkalischen Bereich eine ausreichende Reinigungswirkung entfalten. Unter gebrauchsfertiger Verdünnung wird erfindungsgemäß eine 0,5-100%ige wäßrige Lösung des Reinigungsmittelkonzentrats verstanden. Sofern im Rahmen der vorliegenden Anmeldung pH-Werte einer verdünnten Lösung des Reinigungsmittelkonzentrats gemessen werden, wird als Lösungsmittel vollentsalztes Wasser (VE-Wasser) verwendet. Wenn das Konzentrat mit üblichem Leitungswasser zu einer gebrauchsfertigen Lösung angesetzt wird, können sich je nach Beschaffenheit dieses Wassers geringfügig abweichende pH-Werte ergeben.

Die Einstellung des pH-Werts auf den genannten Bereich erfolgt vorzugsweise durch Zusatz von Säuren und/oder geeigneter Puffersysteme. Bevorzugt ist der Zusatz von wenigstens einer organischen Säure ausgewählt aus der Gruppe bestehend aus Mono-, Di- oder Tricarbonsäuren mit 2 bis 6 C-Atomen. Unter diesen Säuren sind bevorzugt Citronensäure, Weinsäure, Äpfelsäure, Milchsäure, Glykolsäure, Glyoxylsäure, Bernsteinsäure, Adipinsäure und Glutarsäure. Citronensäure ist besonders bevorzugt. Die Säuren werden dem Konzentrat vorzugsweise in einer Menge von 0,5 bis 15 Gew.-%, weiter vorzugsweise 2 bis 10 Gew.-%, zugesetzt.

Erfindungsgemäß kann das Konzentrat wenigstens einen Komplexbildner, insbesondere Chelatbildner, enthalten. Die Komplexbildner dienen der Wasserenthärtung und können durch Komplexieren von Erdalkalionen die Reinigungswirkung gegenüber Kalkseifen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze.

Das erfindungsgemäße Konzentrat kann Nitrilotriessigsäure und/oder ein Salz dieser Säure, besonders bevorzugt deren Trinatriumsalz, enthalten. Dieser NTA-Zusatz bewirkt überraschenderweise besonders gute Kalkseifenentfernung selbst bei niedrigen pH-Werten im Bereich 6 bis 9. Der NTA-Zusatz ist ferner vorteilhaft, wenn das Konzentrat mit stark mineralstoffhaltigem (hartem) Wasser zu einer gebrauchsfertigen Lösung angesetzt werden soll.

Ist aufgrund der Notwendigkeit der Verwendung des Reinigers mit relativ hartem Wasser der Zusatz von Komplexbildnern wie bspw. NTA erforderlich, können u. U. eloxierte Aluminiumoberflächen von der Reinigerlösung angegriffen werden. Überraschenderweise hat sich gezeigt, daß die korrosive Wirkung solcher Komplexbildner vermindert bzw. vollständig vermieden wird durch den Zusatz wenigstens eines Mono- und/oder Diesters der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Besonders bevorzugt ist ein Diester der Phosphorsäure mit Butanol einerseits und Ethylenglykol andererseits. Dieser Ester ist kommerziell unter der Bezeichnung Hordaphos® MDGB erhältlich. Erfindungsgemäß erhält man so eine gute Reinigungswirkung auch bei Verwendung hartem Wassers und trotzdem eine schonende Einwirkung auf exioxierte Aluminiumflächen.

Dem Konzentrat können übliche Konservierungsmittel zugesetzt werden, bspw. p-Hydroxybenzoesäure oder deren Methylester, 5-Brom-5-Nitro-1,3-dioxan, Salicylsäure, 2-Naphtyl-m-N-Dimethylthiocarbanilat, 5-Chlor-5-methyl-4-isothiazolin-3-on, 2-Methyl-4-isothiazolin-3-on sowie Gemische der beiden letztgenannten Verbindungen. Ein bevorzugtes Konservierungsmittel ist p-Hydroxybenzoesäure bzw. deren Methylester. Mit Hilfe dieser Konservierungsmittel läßt sich Mikroben- und Pilzbefall des Reinigungsmittelkonzentrats vermeiden.

Bei Bedarf können Konfektionierhilfsmittel (Lösungsvermittler) zugegeben werden wie bspw. Natriumcumolsulfonat, Natriumtoluolsulfonat, Natriumxylolsulfonat, Harnstoff, Glykole, insbesondere Polypropylenglykole und Polyethylenglykole, Methylacetamid und Fettalkohole, wie bzw. Cetylalkohol.

Die Aufzählung möglicher Inhaltsstoffe ist nicht abschließend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Der Alkanolamingehalt des erfindungsgemäßen Konzentrats beträgt vorzugsweise 10 bis 40 Gew.-%, weiter vorzugsweise 10 bis 20 Gew.-%.

Bevorzugt ist erfindungsgemäß insbesondere die Verwendung des Konzentrats zur maschinell durchgeführten Reinigung in Spülmaschinen.

Ein weiterer Gegenstand der Erfindung ist ein Verfahren zum Reinigen von medizinischen und/oder chirurgischen Instrumenten und/oder Apparaten mit den folgenden Schritten:
a) Aufbringen einer 0,5-100 %igen wäßrigen Lösung eines enzymfreien Reinigungsmittelkonzentrats, das mindestens 10 Gew.-% wenigstens eines Alkanolamins enthält, und das in gebrauchsfertig verdünnter wäßriger Lösung einen pH-Wert von 6 bis 10 auf weist,
b) Einwirkenlassen der Lösung bei einer Temperatur von Raumtemperatur bis Siedetemperatur der Lösung während eines Zeitraums von 2 s bis 10 min,
c) Nachspülen

Das Aufbringen der wäßrigen Lösung des Reinigungsmittelkonzentrats geschieht vorzugsweise durch Sprühen, kann aber bspw. auch durch Eintauchen oder Begießen erfolgen. Das Konzentrat kann in einer sehr hohen Konzentration, ggf. pur, als feiner Nebel aufgesprüht werden und zunächst in dieser hohen Konzentration oder pur einwirken. Anschließend kann ggf. zusätzliches Wasser auf die zu reinigenden Instrumente aufgebracht werden und die so verdünnte Reinigungslösung wird umgewälzt und bspw. durch Besprühen erneut auf die zu reinigenden Instrumente aufgebracht.

Das in Schritt b) definierte Einwirken kann nach dem Aufbringen der Reinigungslösung ein Einwirken in Ruhe, d.h. ohne fortlaufendes Aufbringen bzw. Aufsprühen oder mechanisches Umwälzen bzw. Bewegen der Reinigungslösung umfassen. So kann bspw. insbesondere hochkonzentrierte Reinigungslösung zunächst aufgesprüht werden, nach dem Aufsprühen wird sie dann einwirken gelassen. Der Einwirkzeitraum kann aber auch Abschnitte umfassen, in denen die Reinigungslösung fortlaufend erneut auf die Instrumente aufgebracht bzw. aufgesprüht wird und/oder mechanisch umgewälzt oder auf irgendeine andere Art und Weise bewegt wird. Vorteilhaft ist auch eine Kombination dieser beiden Einwirkarten, also zunächst ein Einwirkenlassen insbesondere hochkonzentrierter Reinigerlösung in Ruhe und anschließendes Verdünnen des Reinigers mit Wasser unter laufender Umwälzung und erneutem Besprühen.

Die wäßrige Lösung des Reinigungsmittelkonzentrats wird vorteilhafterweise als 0,5 bis 20%ige, vorzugsweise als 0,5 bis 10%ige, weiter vorzugsweise als 1 bis 5%ige wäßrige Lösung aufgebracht. Während des Einwirkzeitraumes kann die Lösung des Konzentrats durch zusätzliches Wasser weiter verdünnt werden, dabei sollte aber eine Minimalkonzentration von 0,5% nicht unterschritten werden. Es sei noch angemerkt, daß alle Prozentangaben Gewichtsprozente sind.

Das Einwirkenlassen in Schritt b) geschieht vorzugsweise bei Raumtemperatur bis 55°C, weiter vorzugsweise bei 35 - 50°C, besonders bevorzugt bei 40 - 50°C. Einwirktemperaturen von etwas über 40°C haben sich als besonders vorteilhaft herausgestellt, da dabei einerseits eine gute Reinigungswirkung erzielt wird und andererseits die zu reinigenden Instrumente geschont werden.

Vorteilhafterweise beträgt die Einwirkzeit in Schritt b) 10 s bis 10 min, vorzugsweise 30 s bis 5 min.

Die in Anspruch 12 enthaltene Aufzählung der Verfahrensschritte ist nicht abschließend. Es können zusätzliche Vor- oder Nachspülschritte vorgesehen sein, auch können mehrere Reinigungsschritte hintereinander vorgesehen sein. Bevorzugt ist ferner ein zusätzlicher Desinfektionsschritt, der bspw. durch chemische Desinfektionsmittel erfolgen kann. Besonders bevorzugt ist die Durchführung einer Thermodesinfektion. Diese kann als nachgeschalteter Schritt bspw. mit vollentsalztem Wasser bei 85°C bis 95°C, vorzugsweise 93°C erfolgen. Dieses Wasser kann gleichzeitig zum Nachspülen gemäß Schritt c) verwendet werden, so daß auf diese Weise Nachspülen und Thermodesinfektion miteinander kombiniert werden. Jedoch kann der Thermodesinfektionsschritt statt mit frischem Wasser auch mit der auf die entsprechende Temperatur aufgeheizten Reinigungslösung des Schritts b) erfolgen.

Erfindungsgemäß kann das Reinigungsmittelkonzentrat mit gutem Erfolg im Rahmen eines sogenannten RKI(BGA)-Programms verwendet werden. Bei diesem vom Robert-Koch-Institut (RKI; ehemals Bundesgesundheitsamt (BGA)) anerkannten Programm zur Reinigung und gleichzeitigen Thermodesinfektionen sowohl der zu reinigenden Gegenstände als auch der Reingungslösung wird das Konzentrat in einer Eintankspülmaschine in kaltes einlaufendes Wasser eindosiert. Die kalte Reinigerlösung wird anschließend (bevorzugt unter gleichzeitigem Umwälzen) kontinuierlich auf 93° C aufgeheizt. Die Aufheizzeit ist in erster Linie von der Heizleistung der Maschine abhängig und beträgt bevorzugt zwischen etwa 3 und 15 Minuten. Anschließend erfolgt ein vorzugsweise etwa 10minütiges Einwirken bei 93° C zur Durchführung der Thermodesinfektion. Die heiße Reinigerlösung kann unmittelbar in die Kanalisation abgelassen werden, sie bedarf keiner nachfolgenden Desinfektionsbehandlung. An diesem Reinigungsschritt schließen sich bevorzugt Zwischen- und/oder Nachspülschritte vorzugsweise mit klarem Wasser, ggf. auch unter Zusatz eines Klarspülers, an.

Ggf. können bei Verwendung sogenannter Taktbandspülmaschinen auch mehrere Reinigungsschritte mit dem erfindungsgemäßen Reinigungsmittelkonzentrat hintereinander ausgeführt werden. Dabei ist es bspw. möglich, in einem Reinigungsschritt ein Ultraschalltauchbad, das mit einer wäßrigen Lösung des erfindungsgemäßen Reinigungsmittelkonzentrats gefüllt ist, zu verwenden.

Die Erfindung erzielt eine gute Reinigungswirkung, insbesondere auch in schwer zugänglichen Bereichen chirurgischer Instrumente, bspw. im Gelenkbereich von Scheren.

Die Erfindung wird nachfolgend anhand von Ausführungsbeispielen erläutert.

Erfindungsgemäße Reinigungsmittelkonzentrate werden anhand der Angaben der nachfolgenden Tabelle zubereitet. Die Mengen der einzusetzenden Ausgangsstoffe sind in Gewichtsteilen angegeben.

| | Beispiel 1 | Beispiel 2 | Beispiel 3 | Beispiel 4 |
|---|---|---|---|---|
| Triethanolamin 85% | 15,0 | 15,0 | 15,0 | 15,0 |
| Hordaphos® MDGB¹⁾ | - | - | - | 1,0 |
| Citronensäure | 8,0 | 4,0 | - | 1,2 |
| NTA Trinatriumsalz 40%ige wäßrige Lösung | - | 15,0 | - | 15,0 |
| Noramer® 2000²⁾ | 0,4 | 0,4 | 0,4 | 0,4 |
| p-Hydroxybenzoesäuremethylester | 0,5 | 0,5 | 0,5 | 0,5 |
| N-Acylglutamat | 0,4 | 0,4 | 0,4 | 0,4 |
| Wasser (vollentsalzt) | 75,7 | 64,7 | 83,7 | 66,5 |
| pH-Wert des Konzentrats | 8,5 | 9,6 | 10,1 | 9,4 |
| pH-Wert einer 1%igen wäßrigen Lösung (in VE-Wasser) | 8,0 | 9,8 | 9,8 | 9,9 |

| | | | | |
|---|---|---|---|---|
| ¹⁾Diester der Phosphorsäure mit Butanol und Ethylenglykol | | | | |
| ²⁾Komplexbildner auf Basis von Carboxylat/Sulfonat-Acrylcopolymeren, Firma Norso Haas S.A., Verneuil, En Halatte, Frankreich | | | | |

Die Beispiele 1 und 3 gemäß der Tabelle betreffen komplexbildnerfreie Konzentrate, die sich sehr schonend gegenüber eloxierten Aluminiumoberflächern verhalten. Gegenstand der Beispiele 2 und 4 sind NTA-haltige Konzentrate, die bevorzugt sind beim Ansetzen vom Reinigerlösung mit hartem Wasser. Beispiel 4 enthält zusätzlich einen Phosphorsäureester, der erfindungsgemäß inhibierend auf eloxierte Aluminiumoberflächen wirkt, so daß ein mit diesem Konzentrat angesetzter Reiniger sich gegenüber eloxiertem Aluminium ähnlich schonend verhält wie ein Reiniger gemäß den Beispielen 1 und 3.

Verfahrensbeispiele für maschinelles Reinigen
1. In einer Eintank-Spülmaschine werden die zu reinigenden Instrumente zunächst mit Kaltwasser vorgespült. Anschließend wird die Spülmaschine mit kaltem Wasser gefüllt und das Reinigungsmittelkonzentrat gemäß Beispiel 1 in einer Konzentration von 1,5% zudosiert. Die Reinigungslösung wird auf 40 - 45°C aufgeheizt und 5 min bei dieser Temperatur gehalten. Anschließend wird mit Wasser nachgespült. Zum Schluß erfolgt eine Thermodesinfektion mit vollentsalztem Wasser bei 93°C. Mit diesem Wasser wird gleichzeitig nachgespült.
2. Reinigung nach dem sogenannten RKI(BGA)-Programm
   In eine Eintankspülmaschine wird (ggf. nach einem vorspülgang) kaltes Wasser gefüllt und ein Reinigungsmittelkonzentrat gemäß Beispiel 4 in einer Konzentration von 0,3 bis 1,0 % zudosiert. Die Reinigungslösung wird kontinuierlich in einem Zeitraum von etwa 10 Minuten auf 93° C aufgeheizt und unter Umwälzung etwa 10 Minuten bei dieser Temperatur gehalten. Nach Ablassen der erhitzten Lösung erfolgt eine Zwischenspülung mit kaltem Wasser und in einem letzten Schritt die Schlußspülung, bei der das Wasser (lediglich zum Zwecke des anschließenden schnelleren Trocknens) auf 70° C aufgeheizt wird.
3. Reinigung unter Verwendung einer Taktbandanlage.
   Bei einer Taktbandanlage werden die zu reinigenden Instrumente nacheinander in verschiedene Reinigungskammern gefahren. Bei diesem Verfahrensbeispiel wird eine 3-Kammer-Anlage eingesetzt.
   In der ersten Kammer wird zunächst eine Vorreinigung mit kaltem Wasser für einem Zeitraum von 30 s vorgenommen, anschließend erfolgt eine Reinigung mit einer 1%igen wäßrigen Konzentration des Reinigungsmittelkonzentrats gemäß Beispiel 1 für einen Zeitraum von 5 min bei einer Temperatur von 40°C. Im Anschluß daran wird mit Wasser 30 s lang nachgespült.
   In der zweiten Kammer werden die Instrumente mit einer 2%igen wäßrigen Lösung des Reinigungsmittelkonzentrats gemäß Beispiel 1 bei 35°C 5,5 min in einem Ultraschalltauchbad behandelt.
   In der dritten Kammer erfolgt eine 6-minütige Nachspülung und gleichzeitige Thermodesinfektion mit vollentsalztem Wasser bei 93°C.

## Patentansprüche (Patentansprüche für folgende(n) Vertragsstaat(en): LU, PT)

1. Verwendung eines enzymfreien Reinigungsmittelkonzentrats, das mindestens 10 Gew.-% wenigstens eines Alkanolamins enthält, und das in gebrauchsfertig verdünnter wäßriger Lösung einen pH-Wert von 6 bis 10 aufweist, zur Reinigung medizinischer und/oder chirurgischer Instrumente und/oder Apparate.

2. Verwendung nach Anspruch 1, **dadurch gekennzeichnet, daß** das Alkanolamin folgende Struktur aufweist: 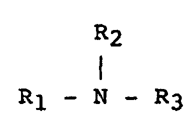 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

3. Verwendung nach Anspruch 2, **dadurch gekennzeichnet, daß** das Konzentrat Mono-, Di- und/oder Triethanolamin enthält.

4. Verwendung nach einem der Ansprüche 1 bis 3, **dadurch gekennzeichnet, daß** das Konzentrat in gebrauchsfertig verdünnter wäßriger Lösung einen pH-Wert von 7 bis 9 aufweist.

5. Verwendung nach einem der Ansprüche 1 bis 4, **dadurch gekennzeichnet, daß** das Konzentrat wenigstens eine organische Säure ausgewählt aus der Gruppe bestehend aus Mono-, Di- oder Tricarbonsäuren mit 2 bis 6 C-Atomen enthält.

6. Verwendung nach Anspruch 5, **dadurch gekennzeichnet, daß** das Konzentrat wenigstens eine Säure ausgewählt aus der Gruppe bestehend aus Citronensäure, Weinsäure, Äpfelsäure, Milchsäure, Glykolsäure, Glyoxylsäure, Bernsteinsäure, Adipinsäure und Glutarsäure enthält.

7. Verwendung nach einem der Ansprüche 1 bis 6, **dadurch gekennzeichnet, daß** das Konzentrat wenigstens einen Komplexbildner enthält.

8. Verwendung nach Anspruch 7, **dadurch gekennzeichnet, daß** das Konzentrat Nitrilotriessigsäure und/oder ein Salz dieser Säure enthält.

9. Verwendung nach Anspruch 7 oder 8, **dadurch gekennzeichnet, daß** das Konzentrat zusätzlich wenigstens einen Mono- und/oder Diester der Phosphorsäure mit aliphatischen Alkoholen der Kettenlänge C₁ bis C₂₂ und/oder aliphatischen Diolen und/oder aliphatischen Polyolen der Kettenlänge C₂ bis C₂₂ enthält.

10. Verwendung nach einem der Ansprüche 1 bis 9, **dadurch gekennzeichnet, daß** der Alkanolamingehalt des Konzentrats 10 bis 40 Gew.-%, vorzugsweise 10 bis 20 Gew.-% beträgt.

11. Verwendung nach einem der Ansprüche 1 bis 10, **dadurch gekennzeichnet, daß** die Reinigung maschinell durchgeführt wird.

12. Verfahren zum Reinigen von medizinischen und/oder chirurgischen Instrumenten und/oder Apparaten, **gekennzeichnet durch** folgende Schritte:
a) Aufbringen einer 0,5-100%igen wäßrigen Lösung eines enzymfreien Reinigungsmittelkonzentrats, das mindestens 10 Gew.-% wenigstens eines Alkanolamins enthält, und das in gebrauchsfertig verdünnter wäßriger Lösung einen pH-Wert von 6 bis 10 aufweist,
b) Einwirkenlassen der Lösung bei einer Temperatur von Raumtemperatur bis zur Siedetemperatur der Lösung während eines Zeitraums von 2 s bis 10 min,
c) Nachspülen.

13. Verfahren nach Anspruch 12, **dadurch gekennzeichnet, daß** das Reinigungsmittelkonzentrat in Schritt a) als 0,5-20%ige, vorzugsweise 0,5-10%ige, weiter vorzugsweise 1-5%ige wäßrige Lösung aufgebracht wird.

14. Verfahren nach Anspruch 12 oder 13, **dadurch gekennzeichnet, daß** das Einwirkenlassen in Schritt b) bei Raumtemperatur bis 55°C, vorzugsweise bei 35 bis 50°C, weiter vorzugsweise bei 40 bis 50°C, geschieht.

15. Verfahren nach einem der Ansprüche 12 bis 14, **dadurch gekennzeichnet, daß** die Einwirkzeit in Schritt b) 10 s bis 10 min, vorzugsweise 30 s bis 5 min beträgt.

16. Verfahren nach einem der Ansprüche 12 bis 15, **dadurch gekennzeichnet, daß** es zusätzlich eine Thermodesinfektion umfaßt.

## Patentansprüche (Patentansprüche für folgende(n) Vertragsstaat(en): AT, BE, CH, LI, DE, DK, ES, FI, FR, GB, GR, IE, IT, NL, SE)

1. Verwendung eines enzymfreien und Mono- und Dialdehyd mit 1 bis 8 C-Atomenfreien Reinigungsmittelkonzentrats, das mindestens 10 Gew.-% wenigstens eines Alkanolamins enthält, und das in gebrauchsfertig verdünnter wäßriger Lösung einen pH-Wert von 6 bis 10 aufweist, zur Reinigung medizinischer und/oder chirurgischer Instrumente und/oder Apparate.

2. Verwendung nach Anspruch 1, **dadurch gekennzeichnet, daß** das Alkanolamin folgende Struktur aufweist: 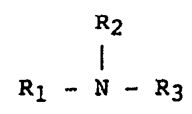 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

3. Verwendung nach Anspruch 2, **dadurch gekennzeichnet, daß** das Konzentrat Mono-, Di- und/oder Triethanolamin enthält.

4. Verwendung nach einem der Ansprüche 1 bis 3, **dadurch gekennzeichnet, daß** das Konzentrat in gebrauchsfertig verdünnter wäßriger Lösung einen pH-Wert von 7 bis 9 aufweist.

5. Verwendung nach einem der Ansprüche 1 bis 4, **dadurch gekennzeichnet, daß** das Konzentrat wenigstens eine organische Säure ausgewählt aus der Gruppe bestehend aus Mono-, Di- oder Tricarbonsäuren mit 2 bis 6 C-Atomen enthält.

6. Verwendung nach Anspruch 5, **dadurch gekennzeichnet, daß** das Konzentrat wenigstens eine Säure ausgewählt aus der Gruppe bestehend aus Citronensäure, Weinsäure, Äpfelsäure, Milchsäure, Glykolsäure, Glyoxylsäure, Bernsteinsäure, Adipinsäure und Glutarsäure enthält.

7. Verwendung nach einem der Ansprüche 1 bis 6, **dadurch gekennzeichnet, daß** das Konzentrat wenigstens einen Komplexbildner enthält.

8. Verwendung nach Anspruch 7, **dadurch gekennzeichnet, daß** das Konzentrat Nitrilotriessigsäure und/oder ein Salz dieser Säure enthält.

9. Verwendung nach Anspruch 7 oder 8, **dadurch gekennzeichnet, daß** das Konzentrat zusätzlich wenigstens einen Mono- und/oder Diester der Phosphorsäure mit aliphatischen Alkoholen der Kettenlänge C₁ bis C₂₂ und/oder aliphatischen Diolen und/oder aliphatischen Polyolen der Kettenlänge C₂ bis C₂₂ enthält.

10. Verwendung nach einem der Ansprüche 1 bis 9, **dadurch gekennzeichnet, daß** der Alkanolamingehalt des Konzentrats 10 bis 40 Gew.-%, vorzugsweise 10 bis 20 Gew.-% beträgt.

11. Verwendung nach einem der Ansprüche 1 bis 10, **dadurch gekennzeichnet, daß** die Reinigung maschinell durchgeführt wird.

12. Verfahren zum Reinigen von medizinischen und/oder chirurgischen Instrumenten und/oder Apparaten, **gekennzeichnet durch** folgende Schritte:
a) Aufbringen einer 0,5-100%igen wäßrigen Lösung eines enzymfreien Reinigungsmittelkonzentrats, das mindestens 10 Gew.-% wenigstens eines Alkanolamins enthält, und das in gebrauchsfertig verdünnter wäßriger Lösung einen pH-Wert von 6 bis 10 aufweist,
b) Einwirkenlassen der Lösung bei einer Temperatur von Raumtemperatur bis zur Siedetemperatur der Lösung während eines Zeitraums von 2 s bis 10 min,
c) Nachspülen.

13. Verfahren nach Anspruch 12, **dadurch gekennzeichnet, daß** das Reinigungsmittelkonzentrat in Schritt a) als 0,5-20%ige, vorzugsweise 0,5-10%ige, weiter vorzugsweise 1-5%ige wäßrige Lösung aufgebracht wird.

14. Verfahren nach Anspruch 12 oder 13, **dadurch gekennzeichnet, daß** das Einwirkenlassen in Schritt b) bei Raumtemperatur bis 55°C, vorzugsweise bei 35 bis 50°C, weiter vorzugsweise bei 40 bis 50°C, geschieht.

15. Verfahren nach einem der Ansprüche 12 bis 14, **dadurch gekennzeichnet, daß** die Einwirkzeit in Schritt b) 10 s bis 10 min, vorzugsweise 30 s bis 5 min beträgt.

16. Verfahren nach einem der Ansprüche 12 bis 15, **dadurch gekennzeichnet, daß** es zusätzlich eine Thermodesinfektion umfaßt.

## Claims (Claims for the following Contracting State(s): LU, PT)

1. Use of an enzyme-free cleaning composition concentrate which comprises at least 10% by weight of at least one alkanolamine and in the ready-to-use dilute aqueous solution has a pH of 6 to 10 for cleaning medical and/or surgical instruments and/or apparatuses.

2. Use according to Claim 1, **characterized in that** the alkanolamine has the following structure: 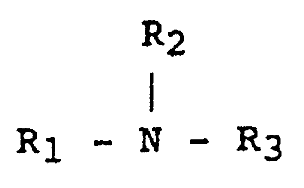 where R₁ is a hydroxyalkyl group having 1 to 6 carbon atoms and R₂ and R₃ independently of one another are the said hydroxyalkyl group or hydrogen.

3. Use according to Claim 2, **characterized in that** the concentrate comprises mono-, di- and/or triethanolamine.

4. Use according to one of Claims 1 to 3, **characterized in that** the concentrate in ready-to-use dilute aqueous solution has a pH of 7 to 9.

5. Use according to one of Claims 1 to 4, **characterized in that** the concentrate comprises at least one organic acid selected from the group consisting of mono-, di- or tricarboxylic acids having 2 to 6 carbon atoms.

6. Use according to Claim 5, **characterized in that** the concentrate comprises at least one acid selected from the group consisting of citric acid, tartaric acid, malic acid, lactic acid, glycolic acid, glyoxylic acids succinic acid, adipic acid and glutaric acid.

7. Use according to one of Claims 1 to 6, **characterized in that** the concentrate comprises at least one complexing agent.

8. Use according to Claim 7, **characterized in that** the concentrate comprises nitrilotriacetic acid and/or a salt of this acid.

9. Use according to Claim 7 or 8, **characterized in that** the concentrate additionally comprises at least one mono- and/or diester of phosphoric acid with aliphatic alcohols of chain length C₁ to C₂₂ and/or aliphatic diols and/or aliphatic polyols of chain length C₂ to C₂₂.

10. Use according to one of Claims 1 to 9, **characterized in that** the alkanolamine content of the concentrate is 10 to 40% by weight, preferably 10 to 20% by weight.

11. Use according to one of Claims 1 to 10, **characterized in that** the cleaning is carried out mechanically.

12. Process for cleaning medical and/or surgical instruments and/or apparatuses, **characterized by** the following steps:
a) applying a 0.5-100% strength aqueous solution of an enzyme-free cleaning composition concentrate which comprises at least 10% by weight of at least one alkanolamine and which in ready-to-use dilute aqueous solution has a pH of 6 to 10,
b) allowing the solution to act at a temperature from room temperature to boiling temperature of the solution for a period of 2 s to 10 min,
c) rinsing.

13. Process according to Claim 12, **characterized in that** the cleaning composition concentrate is applied in step a) as a 0.5-20% strength, preferably 0.5-10% strength, more preferably 1-5% strength, aqueous solution.

14. Process according to Claim 12 or 13, **characterized in that** the action in step b) takes place at room temperature to 55°C, preferably at 35 to 50°C, more preferably at 40 to 50°C.

15. Process according to one of Claims 12 to 14, **characterized in that** the time of action in step b) is 10 s to 10 min, preferably 30 s to 5 min.

16. Process according to one of Claims 12 to 15, **characterized in that** it additionally includes a thermal disinfection.

## Claims (Claims for the following Contracting State(s): AT, BE, CH, LI, DE, DK, ES, FI, FR, GB, GR, IE, IT, NL, SE)

1. Use of an enzyme-free cleaning composition concentrate which is free of mono- and dialdehydes having 1 to 8 carbon atoms and which comprises at least 10% by weight of at least one alkanolamine and in the ready-to-use dilute aqueous solution has a pH of 6 to 10 for cleaning medical and/or surgical instruments and/or apparatuses.

2. Use according to Claim 1, **characterized in that** the alkanolamine has the following structure: 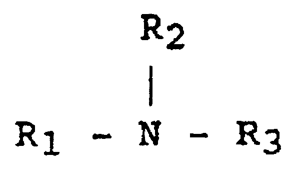 where R₁ is a hydroxyalkyl group having 1 to 6 carbon atoms and R₂ and R₃ independently of one another are the said hydroxyalkyl group or hydrogen.

3. Use according to Claim 2, **characterized in that** the concentrate comprises mono-, di- and/or triethanolamine.

4. Use according to one of Claims 1 to 3, **characterized in that** the concentrate in ready-to-use dilute aqueous solution has a pH of 7 to 9.

5. Use according to one of Claims 1 to 4, **characterized in that** the concentrate comprises at least one organic acid selected from the group consisting of mono-, di- or tricarboxylic acids having 2 to 6 carbon atoms.

6. Use according to Claim 5, **characterized in that** the concentrate comprises at least one acid selected from the group consisting of citric acid, tartaric acid, malic acid, lactic acid, glycolic acid, glyoxylic acid, succinic acid, adipic acid and glutaric acid.

7. Use according to one of Claims 1 to 6, **characterized in that** the concentrate comprises at least one complexing agent.

8. Use according to Claim 7, **characterized in that** the concentrate comprises nitrilotriacetic acid and/or a salt of this acid.

9. Use according to Claim 7 or 8, **characterized in that** the concentrate additionally comprises at least one mono- and/or diester of phosphoric acid with aliphatic alcohols of chain length C₁ to C₂₂ and/or aliphatic diols and/or aliphatic polyols of chain length C₂ to C₂₂.

10. Use according to one of Claims 1 to 9, **characterized in that** the alkanolamine content of the concentrate is 10 to 40% by weight, preferably 10 to 20% by weight.

11. Use according to one of Claims 1 to 10, **characterized in that** the cleaning is carried out mechanically.

12. Process for cleaning medical and/or surgical instruments and/or apparatuses, **characterized by** the following steps:
a) applying a 0.5-100% strength aqueous solution of an enzyme-free cleaning composition concentrate which comprises at least 10% by weight of at least one alkanolamine and which in ready-to-use dilute aqueous solution has a pH of 6 to 10,
b) allowing the solution to act at a temperature from room temperature to boiling temperature of the solution for a period of 2 s to 10 min,
c) rinsing.

13. Process according to Claim 12, **characterized in that** the cleaning composition concentrate is applied in step a) as a 0.5-20% strength, preferably 0.5-10% strength, more preferably 1-5% strength, aqueous solution.

14. Process according to Claim 12 or 13, **characterized in that** the action in step b) takes place at room temperature to 55°C, preferably at 35 to 50°C, more preferably at 40 to 50°C.

15. Process according to one of Claims 12 to 14, **characterized in that** the time of action in step b) is 10 s to 10 min, preferably 30 s to 5 min.

16. Process according to one of Claims 12 to 15, **characterized in that** it additionally includes a thermal disinfection.

## Revendications (Revendications pour l'(les) Etat(s) contractant(s) suivant(s): LU, PT)

1. Utilisation d'un détergent concentré sans enzymes, qui contient au moins 10 % en poids d'au moins une alcanolamine, et qui présente, en solution diluée prête à l'emploi, un pH compris entre 6 et 10, pour le nettoyage d'instruments et/ou d'appareils médicaux et/ou chirurgicaux.

2. Utilisation selon 1a revendication 1, **caractérisée en ce que** l'alcanolamine présente la structure suivante: 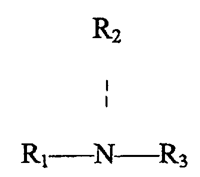 où R₁ est un groupe hydroxy-alkyle à 1 à 6 atomes de C et où R₂ et R₃, indépendamment l'un de l'autre, peuvent être le groupe hydroxy-alkyle susmentionné ou des atomes d'hydrogène.

3. Utilisation selon la revendication 2, **caractérisée en ce que** le concentré contient de la mono-, de la di- et/ou de la triéthanolamine.

4. Utilisation selon une des revendications 1 à 3, **caractérisée en ce que** la solution aqueuse diluée prête à l'utilisation du concentré présente un pH compris entre 7 et 9.

5. Utilisation selon une des revendications 1 à 4, **caractérisée en ce que** le concentré contient au moins un acide organique choisi dans le groupe des acides mono-, di- ou tricarboxyliques avec 2 à 6 atomes de C.

6. Utilisation selon la revendication 5, **caractérisée en ce que** le concentré contient au moins un acide choisi dans le groupe qui consiste en l'acide citrique, l'acide tartrique, l'acide malique, l'acide lactique, l'acide glycolique, l'acide glyoxylique, l'acide succinique, l'acide adipique et l'acide glutarique.

7. Utilisation selon une des revendications 1 à 6, **caractérisée en ce que** le concentré contient au moins un complexant.

8. Utilisation selon la revendication 7, **caractérisée en ce que** le concentré contient de l'acide nitrilo-triacétique et/ou un sel de cet acide.

9. Utilisation selon une des revendications 7 ou 8, **caractérisée en ce que** le concentré contient en plus au moins un mono- ou un diester de l'acide phosphorique avec des alcools aliphatiques de longueur de chaîne C₁ à C₂₂ et/ou avec des diols et/ou des polyols aliphatiques de longueur de chaîne C₂ à C₂₂.

10. Utilisation selon une des revendications 1 à 9, **caractérisée en ce que** la teneur en alcanolamine du concentré est de 10 à 40 % en poids, de préférence 10 à 20 % en poids.

11. Utilisation selon une des revendications 1 à 10, **caractérisée en ce que** le nettoyage s'effectue dans une machine.

12. Procédé de nettoyage d'instruments et/ou d'appareils médicaux et/ou chirurgicaux, **caractérisé par** les étapes suivantes :
a) Appliquer une solution aqueuse entre 0,5 et 100 % d'un concentré détergent sans enzymes, qui contient au moins 10 % en poids d'au moins une alcanolamine, et qui, en solution aqueuse diluée prête à l'emploi, présente un pH compris entre 6 et 10,
b) Laisser agir la solution à une température comprise entre la température ambiante et la température d'ébullition de la solution pendant un intervalle de temps de 2 s à 10 min.
c) Rincer.

13. Procédé selon la revendication 12, **caractérisé en ce que** le concentré détergent est appliqué à l'étape a) sous forme d'une solution aqueuse entre 0,5 et 20 %, de préférence entre 0,5 et 10 %, encore de préférence entre 1 et 5 %.

14. Procédé selon une des revendications 12 ou 13, **caractérisé en ce que** la phase d'action de la solution à l'étape b) est effectuée entre la température ambiante et 55 °C, de préférence entre 35 et 50 °C, encore de préférence entre 40 et 50 °C.

15. Procédé selon une des revendications 12 à 14, **caractérisé en ce que** la durée de 1a phase d'action à l'étape b) est comprise entre 10 s et 10 min, de préférence entre 30 s et 5 min.

16. Procédé selon une des revendications 12 à 15, **caractérisé en ce qu'**il comprend en plus une désinfection thermique.

## Revendications (Revendications pour l'(les) Etat(s) contractant(s) suivant(s): AT, BE, CH, LI, DE, DK, ES, FI, FR, GB, GR, IE, IT, NL, SE)

1. Utilisation d'un détergent concentré sans enzymes et exempt de mono- et de di-aldéhydes avec 1 à 8 atomes de C, qui contient au moins 10 % en poids d'au moins une alcanolamine, et qui présente, en solution diluée prête à l'emploi, un pH compris entre 6 et 10, pour le nettoyage d'instruments et/ou d'appareils médicaux et/ou chirurgicaux.

2. Utilisation selon la revendication 1, **caractérisée en ce que** l'alcanolamine présente la structure suivante : 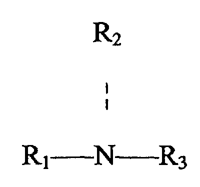 où R₁ est un groupe hydroxy-alkyle à 1 à 6 atomes de C et où R₂ et R₃, indépendamment l'un de l'autre, peuvent être le groupe hydroxy-alkyle susmentionné ou des atomes d'hydrogène.

3. Utilisation selon la revendication 2, **caractérisée en ce que** le concentré contient de la mono-, de la di- et/ou de la triéthanolamine.

4. Utilisation selon l'une des revendications 1 à 3, **caractérisée en ce que** la solution aqueuse diluée prête à l'utilisation du concentré présente un pH compris entre 7 et 9.

5. Utilisation selon l'une des revendications 1 à 4, **caractérisée en ce que** le concentré contient au moins un acide organique choisi dans le groupe des acides mono-, di- ou tricarboxyliques avec 2 à 6 atomes de C.

6. Utilisation selon la revendication 5, **caractérisée en ce que** le concentré contient au moins un acide choisi dans le groupe qui consiste en l'acide citrique, l'acide tartrique, l'acide malique, l'acide lactique, l'acide glycolique, l'acide glyoxylique, l'acide succinique, l'acide adipique et l'acide glutarique.

7. Utilisation selon l'une des revendications 1 à 6, **caractérisée en ce que** le concentré contient au moins un complexant.

8. Utilisation selon la revendication 7, **caractérisée en ce que** le concentré contient de l'acide nitrilo-triacétique et/ou un sel de cet acide.

9. Utilisation selon l'une des revendications 7 ou 8, **caractérisée en ce que** le concentré contient en plus au moins un mono- ou un diester de l'acide phosphorique avec des alcools aliphatiques de longueur de chaîne C₁ à C₂₂ et/ou avec des diols et/ou des polyols aliphatiques de longueur de chaîne C₂ à C₂₂.

10. Utilisation selon l'une des revendications 1 à 9, **caractérisée en ce que** la teneur en alcanolamine du concentré est de 10 à 40 % en poids, de préférence 10 à 20 % en poids.

11. Utilisation selon l'une des revendications 1 à 10, **caractérisée en ce que** le nettoyage s'effectue dans une machine.

12. Procédé de nettoyage d'instruments et/ou d'appareils médicaux et/ou chirurgicaux, **caractérisé par** les étapes suivantes :
a) Appliquer une solution aqueuse entre 0,5 et 100 % d'un concentré détergent sans enzymes, qui contient au moins 10 % en poids d'au moins une alcanolamine, et qui, en solution aqueuse diluée prête à l'emploi, présente un pH compris entre 6 et 10,
b) Laisser agir la solution à une température comprise entre la température ambiante et la température d'ébullition de la solution pendant un intervalle de temps de 2 s à 10 min.
c) Rincer.

13. Procédé selon la revendication 12, **caractérisé en ce que** le concentré détergent est appliqué à l'étape a) sous forme d'une solution aqueuse entre 0,5 et 20 %, de préférence entre 0,5 et 10 %, encore de préférence entre 1 et 5%.

14. Procédé selon l'une des revendications 12 ou 13, **caractérisé en ce que** la phase d'action de la solution à l'étape b) est effectuée entre la température ambiante et 55 °C, de préférence entre 35 et 50 °C, encore de préférence entre 40 et 50 °C.

15. Procédé selon l'une des revendications 12 à 14, **caractérisé en ce que** la durée de la phase d'action à l'étape b) est comprise entre 10 s et 10 min, de préférence entre 30 s et 5 min.

16. Procédé selon l'une des revendications 12 à 15, **caractérisé en ce qu'**il comprend en plus une désinfection thermique.
